# EUROPÄISCHE PATENTANMELDUNG

(11) **EP 1 166 748 A2**
(43) Veröffentlichungstag der Anmeldung: **02.01.2002**
(21) Anmeldenummer: 01106292.4
(22) Anmeldetag: 15.03.2001
(51) Int. Cl.: A61K 7/13

(54) **Mittel und Verfahren zur Färbung keratinischer Fasern**

(30) Priorität: 29.06.2000 DE 10031695
(71) Anmelder: Wella Aktiengesellschaft, 64274 Darmstadt (DE)
(72) Erfinder: Braun, Petra, 64839 Münster (DE); Bahnmüller, Iris, 69469 Weinheim (DE)

(57) **Zusammenfassung**

Gegenstand der vorliegenden Erfindung ist ein Mittel zum Färben von Keratinfasern, welches vor der Anwendung nicht mit einem Oxidationsmittel vermischt wird und dadurch gekennzeichnet ist, dass es frei von Magansalzen ist und in einem geeigneten kosmetischen Träger eine in Gegenwart von Luftsauerstoff zur Färbung von Keratinfasern geeignete Kombination aus
(a) mindestens einem 4,5-Diaminopyrazol-Derivat der allgemeinen Formel (I) oder dessen physiologisch verträglichen Salzen, 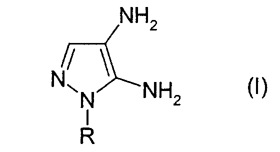 und
(b1) mindestens ein 5-Amino-2-methylphenolderivat gemäß der allgemeinen Formel (II) oder dessen physiologisch verträglichen Salzen, 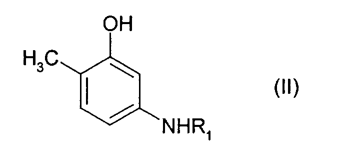 und/oder
(b2) mindestens ein m-Phenylendiaminderivat gemäß der allgemeinen Formel (III) oder dessen physiologisch verträglichen Salzen, 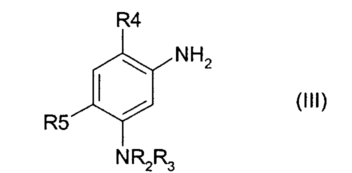 enthält; sowie ein Verfahren zum Färben von Keratinfasern unter Verwendung dieses Mittels.

## Beschreibung

Die vorliegende Erfindung betrifft Zusammensetzungen zur Färbung keratinischer Fasern, insbesonders menschlicher Haare, auf der Basis eines 4,5-Diaminopyrazols und einer Kupplersubstanz, welche ohne Zusatz eines Oxidatonsmittels beständige Färbungen ergeben.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Für bestimmte Anwendungen besteht jedoch ein Bedarf nach Färbemitteln, welche in Gegenwart von Luftsauerstoff auch ohne den Zusatz von Oxidationsmitteln beständige Färbungen ergeben.

Die Synthese von 4,5-Diaminopyrazolen und deren Verwendung in vor der Anwendung mit einem Oxidationsmittel zu vermischenden Oxidationshaarfärbemitteln ist aus der EP-B 0 375 977 bekannt. Weiterhin ist es aus der EP-A 0 749 748 bekannt, bei Haarfärbungen mit an der Luft ohne Zusatz eines Katalysators nicht bzw. nur schwer oxidierbaren o-Diaminopyrazolen zur Katalysierung der Luftoxidation Mangansalze einzusetzen. Ebenfalls ist es aus der EP-A 0 740 931 bekannt, dass bestimmte in 3-Stellung substituierte 4,5-Diaminopyrazole auch ohne den Zusatz von Oxidationsmitteln oder Katalysatoren in Gegenwart von Luftsauerstoff zur Färbung von Keratinfasern geeignet sind. Diese Mittel sind jedoch nicht in jeder Hinsicht befriedigend.

Es bestand daher weiterhin ein Bedarf nach Färbemitteln, welche ohne den Zusatz von Metallkatalysatoren oder/und Oxidationsmitteln in Gegenwart von Luftsauerstoff auf schonende Weise intensive Färbungen von Keratinfasern ergeben.

Gegenstand der vorliegenden Anmeldung ist ein Mittel zum Färben von Keratinfasern, das vor der Anwendung nicht mit einem Oxidationsmittel vermischt wird und dadurch gekennzeichnet ist, dass es frei von Mangansalzen ist und in einem geeigneten kosmetischen Träger eine in Gegenwart von Luftsauerstoff zur Färbung von Keratinfasern geeignete Kombination aus
(a) mindestens einem 4,5-Diaminopyrazol-Derivat der allgemeinen Formel (I) oder dessen physiologisch verträglichen Salzen, 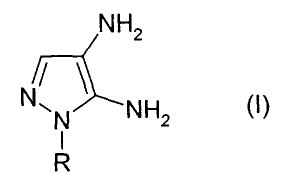 worin **R** Wasserstoff, eine geradkettige oder verzweigte Alkylgruppe mit 1 bis 6 Kohlenstoffatomen, eine geradkettige oder verzweigte Monohydroxyalkylgruppe mit 1 bis 6 Kohlenstoffatomen, eine geradkettige oder verzweigte Polyhydroxyalkylgruppe mit 2 bis 6 Kohlenstoffatomen, eine geradkettige oder verzweigte Monoaminoalkylgruppe mit 1 bis 6 Kohlenstoffatomen, eine geradkettige oder verzweigte Polyaminoalkylgruppe mit 2 bis 6 Kohlenstoffatomen, eine mit einem Halogenatom monosubstituierte Benzylgruppe, eine mit einer geradkettigen oder verzweigten Alkylgruppe mit 1 bis 6 Kohlenstoffatomen monosubstituierte Benzylgruppe, eine mit einer geradkettigen oder verzweigten Alkoxygruppe mit 1 bis 6 Kohlenstoffatomen monosubstituierte Benzylgruppe oder eine unsubstituierte Benzylgruppe darstellt; und
(b) mindestens einer Verbindung, welche ausgewählt ist aus
   (b1) 5-Amino-2-methylphenolderivaten der allgemeinen Formel (II) oder deren physiologisch verträglichen Salzen, 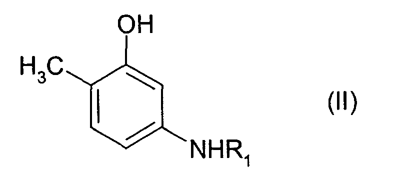 worin **R1** gleich Wasserstoff, einer geradkettigen oder verzweigten Alkylgruppe mit 1 bis 4 Kohlenstoffatomen, einer geradkettigen oder verzweigten Monohydroxyalkylgruppe mit 2 bis 4 Kohlenstoffatomen oder einer geradkettigen oder verzweigten Polyhydroxyalkylgruppe mit 3 bis 4 Kohlenstoffatomen ist; und
   (b2) m-Phenylendiaminderivaten der allgemeinen Formel (III) oder deren physiologisch verträglichen Salzen, 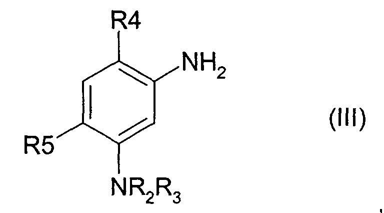 worin **R2** und **R3** unabhängig voneinander ein Wasserstoffatom, eine geradkettige oder verzweigte Alkylgruppe mit 1 bis 4 Kohlenstoffatomen, eine geradkettige oder verzweigte Monohydroxyalkylgruppe mit 2 bis 4 Kohlenstoffatomen oder eine geradkettige oder verzweigte Polyhydroxyalkylgruppe mit 3 bis 4 Kohlenstoffatomen bedeuten, und
      **R4** und **R5** unabhängig voneinander ein Wasserstoffatom, eine geradkettige oder verzweigte Alkylgruppe mit 1 bis 6 Kohlenstoffatomen, eine geradkettige oder verzweigte Alkoxygruppe mit 1 bis 4 Kohlenstoffatomen, eine geradkettige oder verzweigte Monohydroxyalkoxygruppe mit 2 bis 4 Kohlenstoffatomen, eine geradkettige oder verzweigte Polyhydroxyalkoxygruppe mit 3 bis 4 Kohlenstoffatomen, eine geradkettige oder verzweigte Monohydroxyalkylgruppe mit 2 bis 4 Kohlenstoffatomen, eine geradkettige oder verzweigte Polyhydroxyalkylgruppe mit 3 bis 4 Kohlenstoffatomen oder eine 2,4-Diaminophenoxyalkoxy-Gruppe mit 1 bis 4 Kohlenstoffatomen in der Alkylkette bedeuten; enthält.

Bevorzugte Verbindungen der allgemeinen Formel (I) sind 4,5-Diamino-1-methyl-1 H-pyrazol; 4,5-Diamino-1-(4'-methylbenzyl)-pyrazol; 4,5-Diamino-1-(2'-hydroxyethyl)-1 H-pyrazol; 4,5-Diamino-1-benzyl-1 H-pyrazol; 4,5-Diamino-1-ethyl-1 H-pyrazol; 4,5-Diamino-1-isopropyl-1 H-pyrazol; 4,5-Diamino-1-(4'-methoxybenzyl)-1 H-pyrazol; 4,5-Diamino-1 H-pyrazol; 4,5-Diamino-1-(3'-methoxybenzyl)-1H-pyrazol und 4,5-Diamino-1-(4'-chlorbenzyl)-1 H-pyrazol, oder deren physiologisch verträgliche Salze.

Bevorzugte Verbindungen der allgemeinen Formel (II) sind 5-Amino-2-methylphenol, 5-Methylamino-2-methylphenol und 5-[(2'-Hydroxyethyl)-amino]-2-methylphenol sowie deren physiologisch verträgliche Salze.

Bevorzugte Verbindungen der allgemeinen Formel (III) sind 2,4-Diamino-1-(2'-hydroxyethoxy)benzol, 2,4-Diamino-1-(2',3'-dihydroxypropoxy)-benzol, 1,3-Bis(2',4'-diaminophenoxy)propan, 2-Amino-4-[(2'-hydroxyethyl)amino]anisol, 1,5-Bis(2'-hydroxyethoxy)-2,4-diaminobenzol und m-Phenylendiamin sowie deren physiologisch verträgliche Salze.

Zur Abrundung des Farbergebnisses sowie zur Erzeugung von speziellen Farbeffekten können der vorgenannten Farbstoffkombination weitere übliche Oxidationsfarbstoffvorstufen, beispielsweise Derivate des p-Phenylendiamins wie zum Beispiel 2-(2',5'-Diaminophenyl)ethanol; Resorcinderivate wie zum Beispiel Resorcin oder 4-Chlorresorcin; Amino-und Hydroxyderivate des 1,3-Benzodioxols; Naphthalinderivate wie zum Beispiels 1-Hydroxynaphthalin, 1,5- Dihydroxynaphthalin oder 1,7-Dihydroxynaphthalin; sowie übliche direktziehende Farbstoffe aus der Gruppe der sauren oder basischen Farbstoffe, Triphenylmethanfarbstoffe, Anthra-chinonfarbstoffe, aromatischen Nitrofarbstoffe, Azofarbstoffe, Lebensmittelfarbstoffe oder Dispersionsfarbstoffe; oder weitere Farbkomponenten, beispielsweise 6-Amino-2-methylphenol und 2-Amino-5-methylphenol, zugesetzt werden. Das Färbemittel kann diese Farbkomponenten in einer Menge von etwa 0,1 bis 4,0 Gewichtsprozent enthalten.

Selbstverständlich können die genannten Farbstoffe und Farbstoffvorstufen, sofern es Basen sind, auch in Form der physiologisch verträglichen Salze mit organischen oder anorganischen Säuren, wie beispielsweise Salzsäure oder Schwefelsäure, beziehungsweise -sofern sie aromatische OH-Gruppen besitzen- in Form der Salze mit Basen, zum Beispiel als Alkaliphenolate, eingesetzt werden.

Die Gesamtkonzentration an Farbvorstufen beträgt 0,1 bis 10 Gewichts-prozent, bevorzugt 0,2 bis 6 Gewichtsprozent. Die Konzentration der einzelnen Haarfarbstoffe beträgt 0,01 bis 5 Gewichtsprozent, vorzugsweise 0,1 bis 4 Gewichtsprozent.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, UV-Absorber, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichts-prozent, die Verdicker in einer Menge von etwa 0,1 bis 25 Gewichts-prozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen schwach sauren bis alkalischen pH-Wert von etwa 6,5 bis 11,5, vorzugsweise etwa 8 bis 10, auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen hingegen anorganische oder organische Säuren, wie zum Beispiel Phosphorsäure, Essigsäure, Zitronensäure oder Weinsäure, in Betracht.

Für die Anwendung zur Färbung von Haaren bringt man die vorstehend beschriebene Farbträgermasse ohne vorherigen Zusatz eines Oxidationsmittels auf das Haar auf, wobei je nach Haarfülle im allgemeinen eine Menge von etwa 60 bis 200 Gramm verwendet wird. Sodann lässt man das Färbemittel bei 15 bis 50 °C etwa 5 bis 45 Minuten lang, vorzugsweise 10 Minuten lang, auf das Haar einwirken, spült anschliessend das Haar mit Wasser und trocknet es. Gegebenenfalls wird im Anschluss an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die nachfolgenden Beispiele sollen den Gegenstand näher erläutern, ohne ihn auf diese Beispiele zu beschränken:

### Beispiele

Die in den nachfolgenden Tabellen 1 bis 14 verwendeten Kurzbezeichnungen haben folgende Bedeutung. Die Mengenangaben in den Tabellen 1 bis 14 sind jeweils in Gramm.

| **A. Kupplersubstanzen** | |
|---|---|
| K11 | 1,3-Diaminobenzol |
| K12 | 2-Amino-4-(2'-hydroxyethyl)amino-anisol-sulfat |
| K13 | 2,4-Diamino-1-(2'-hydroxyethoxy)benzol-sulfat |
| K14 | 2,4-Diamino-5-fluor-toluol-sulfat |
| K15 | 3-Amino-2-methylamino-6-methoxy-pyridin |
| K16 | 3,5-Diamino-2,6-dimethoxy-pyridin-Dihydrochlorid |
| K17 | 2,4-Diamino-5-ethoxy-toluol-sulfat |
| K18 | N-(3-Dimethylamino)phenylharnstoff |
| K19 | 1,3-Bis(2,4-diaminophenoxy)propan-Tetrahydrochlorid |
| K21 | 3-Amino-phenol |
| K22 | 5-Amino-2-methyl-phenol |
| K23 | 5-Amino-6-chlor-2-methyl-phenol |
| K24 | 5-Amino-4-fluor-2-methyl-phenol-sulfat |
| K25 | 1-Naphthol |
| K26 | 1-Acetoxy-2-methyl-naphthalin |
| K31 | Resorcin |
| K32 | 2-Methyl-resorcin |
| K33 | 4-Chlor-resorcin |
| K34 | 4-(2'-Hydroxyethyl)amino-1,2-methylendioxybenzol-Hydrochlorid |
| K35 | 3,4-Methylendioxy-phenol |
| K36 | 2-Amino-5-methyl-phenol |

| **B. Entwicklersubstanz** | |
|---|---|
| E1 | 2,5-Diaminotoluol-sulfat |
| E2 | 1,4-Diaminobenzol |
| E3 | 2,5-Diamino-phenylethanol-sulfat |
| E7 | N,N-Bis(2'-hydroxyethyl)-p-phenylendiamin-sulfat |
| | |
| E8 | 4,5-Diamino-1-(2'-hydroxyethyl)-pyrazol-sulfat |

| **C. direktziehende Farbstoffe** | |
|---|---|
| D1 | 2,6-Diamino-3-((pyridin-3-yl)azo)pyridin |
| D2 | 6-Chlor-2-ethylamino-4-nitro-phenol |
| D3 | 2-Amino-6-chlor-4-nitro-phenol |

### Beispiele 1 bis 8: Haarfärbelösungen mit alkalischem pH-Wert

Es wird folgende Farbelösung hergestellt:

| | |
|---|---|
| 10,0 g | Isopropanol |
| 10,0 g | Laurylalkoholdiglykolether-sulfat-Natriumsalz, (28-prozentige wässrige Lösung) |
| 10,0 g | Ammoniak 25-prozentige wässrige Lösung |
| 0,1 g | Ascorbinsäure |
| X g | Farbvorstufen gemäß Tabelle 1 |
| ad 100 g | Wasser, vollentsalzt |

Die Haarfärbelösungen weisen einen pH-Wert von 9 bis 10,5 auf.

Zur Anwendung werden 60 g der Haarfärbelösung auf die Haare aufgetragen. Nach einer Einwirkungszeit von 10 Minuten bei 40 °C werden die Haare mit Wasser gespült, shampooniert und getrocknet.
Die Färbeergebnisse sind in Tabelle 1 zusammengefasst.

**Tabelle 1:**

| **Beispiele 1 - 8** | | | | | | | | |
|---|---|---|---|---|---|---|---|---|
| **Bsp.** | **1** | **2** | **3** | **4** | **5** | **6** | **7** | **8** |
| **Farb-stoffe** | | | | | | | | |
| E8 | 1,72 | 1,21 | 1,85 | 1,39 | 1,2 | 1,2 | 0,96 | 2,4 |
| K12 | | | 0,42 | | 0,28 | | 0,84 | |
| K14 | | | | 0,26 | | | | 0,26 |
| K18 | 0,40 | | | 0,45 | | | | 0,45 |
| K21 | 0,30 | 0,1 | 0,52 | | 0,44 | | | |
| K22 | | 0,38 | | | | | 0,25 | |
| K25 | | | 0,22 | 0,22 | | | 0,02 | 0,22 |
| K31 | 0,30 | 0,13 | | | | | | |
| K32 | 0,30 | | | | | | | |
| K36 | | | | | | 0,62 | | 0,62 |
| | | | | | | | | |
| **Farbe** | rötliches beige | orange | rotblau | helles aubergine | rotblau | rot | rötliches beige | helles blaurot |
| **Nach einer Woche** | rötliches beige | orange | rotblau | helles aubergine | rotblau | rot | warmes braun | blaurot |

### Beispiele 9 bis 15: Haarfärbemittel in Cremeform

| | |
|---|---|
| 15,00 g | Cetylalkohol |
| 3,50 g | Laurylalkoholdiglykolether-sulfat-Natriumsalz, (28-prozentige wässrige Lösung) |
| 3,00 g | Ammoniak, 25prozentige wässrige Lösung |
| 0,10g | Ascorbinsäure |
| X g | Farbvorstufen gemäß Tabelle 2 |
| ad 100,00 g | Wasser |

50 g dieses Haarfärbemittels werden auf blonde Naturhaare aufgetragen. Das Haar wird nach einer Einwirkungszeit von 15 Minuten bei 40 °C mit Wasser gespült und getrocknet. Die Färbeergebnisse sind in Tabelle 2 zusammengefasst.

**Tabelle 2:**

| **Beispiele 9 - 15** | | | | | | | |
|---|---|---|---|---|---|---|---|
| **Bsp.** | **9** | **10** | **11** | **12** | **13** | **14** | **15** |
| **Farb- stoffe** | | | | | | | |
| E8 | 2,15 | 1,07 | 1,07 | 1,07 | 1,07 | 1,08 | 1,08 |
| K12 | | | 0,42 | 0,28 | 0,28 | 1,12 | |
| K13 | | | | 0,72 | 0,72 | | |
| K14 | | | | 0,26 | | | |
| K18 | | 0,54 | | | | | 0,45 |
| K22 | | 0,25 | | | | 0,12 | |
| K24 | | | 0,38 | | 0,19 | | |
| K25 | | | 0,22 | | | | |
| K31 | 0,88 | | | | | | |
| K32 | 0,25 | | | | | | 0,28 |
| **Farbe** | helles orange | helles orange | rötliches beige | gelb-braun | bläuliches beige | bläuliches beige | hell flieder-farben |
| **Nach einer Woche** | helles orange | helles braunrot | warmes braun | mahagonie-farben | hell kastanien-farben | hell kastanien-farben | flieder-farben |

### Beispiele 16 bis 80: Haarfärbemittel in Gelform

| | |
|---|---|
| 0,40 g | Natriumhydroxid, fest |
| 0,15 g | Ascorbinsäure |
| 7,00 g | Isopropanol |
| 15,00 g | Ölsäure |
| 10,00 g | Ammoniak, 25prozentige wässrige Lösung |
| X g | Farbstoffvorstufen gemäß Tabelle 3 bis 14 |
| ad 100,00 g | Wasser |

70 g des Haarfärbemittels werden 15 Minuten lang auf blonde mensch-liche Haare einwirken gelassen. Danach wird mit Wasser gespült und sodann getrocknet.

Die Färbeergebnisse sind in den nachfolgenden Tabellen 3 bis 14 zusammengefasst.

**Tabelle 3:**

| **Beispiele 16 - 22** | | | | | | | |
|---|---|---|---|---|---|---|---|
| **Bsp.** | **16** | **17** | **18** | **19** | **20** | **21** | **22** |
| **Farb- stoffe** | | | | | | | |
| E8 | 1,21 | 1,08 | 1,08 | 1,21 | 1,21 | 1,21 | 1,21 |
| K12 | | | 0,28 | | | | |
| K13 | 0,72 | | | 0,72 | 0,48 | 0,27 | |
| K14 | 0,26 | | | | | | |
| K18 | | | 0,45 | | | | |
| K19 | 0,28 | | | 0,28 | | | 0,84 |
| K21 | | 0,11 | | | | 0,44 | |
| K22 | | 0,37 | | | 0,22 | | 0,25 |
| K24 | | | | 0,19 | | | |
| K25 | | | 0,22 | | 0,22 | | 0,02 |
| K32 | | 0,11 | | | | | |
| | | | | | | | |
| **Farbe** | helles blaurot | orange | helles aubergine | bläuliches beige | rötliches beige | rotblau | rötliches beige |
| **Nach einer Woche** | mahagoni-farben | orange | helles aubergine | hell kastanien-farben | warmes braun | rotblau | warmes braun |

**Tabelle 4:**

| **Beispiele 23 - 29** | | | | | | | |
|---|---|---|---|---|---|---|---|
| **Bsp.** | **23** | **24** | **25** | **26** | **27** | **28** | **29** |
| **Farb- stoffe** | | | | | | | |
| E8 | 1,21 | 1,21 | 1,21 | 0,96 | 0,96 | 0,96 | 0,72 |
| K12 | | | | | 0,8 | | |
| K14 | | | | 0,81 | | | |
| K18 | | 0,45 | 0,45 | | | 0,54 | |
| K19 | 1,12 | | | 0,28 | 0,28 | | 1,12 |
| K22 | 0,12 | | 0,62 | | | | |
| K24 | | | | | 0,19 | 0,25 | 0,12 |
| K25 | | | 0,22 | | | | |
| K31 | | 0,28 | | | | | |
| | | | | | | | |
| **Farbe** | bläuliches beige | beige | helles orange | bläuliches beige | bläuliches beige | helles orange | bläuliches beige |
| **Nach einer Woche** | hell kastanien-farben | hellrotes beige | helles orange | hell kastanien-farben | hell kastanien-farben | helles braunrot | hell kastanien-farben |

**Tabelle 5:**

| **Beispiele 30 - 36** | | | | | | | |
|---|---|---|---|---|---|---|---|
| **Bsp.** | **30** | **31** | **32** | **33** | **34** | **35** | **36** |
| **Farb- stoffe** | | | | | | | |
| E8 | 0,96 | 1,08 | 1,08 | 1,08 | 2,64 | 1,2 | 1,2 |
| K11 | | | | | | | 0,23 |
| K14 | 0,26 | | | | 0,26 | | |
| K18 | | | | | 0,45 | | |
| K19 | | 0,28 | 0,84 | | 0,52 | 0,92 | |
| K21 | | 0,44 | | 0,11 | | | |
| K24 | 0,38 | | 0,25 | 0,37 | | | |
| K25 | 0,22 | | 0,02 | | 0,22 | | |
| K31 | | | | 0,11 | | | |
| K32 | | | | | | 0,19 | 0,19 |
| K34 | | | | | | 0,33 | 0,33 |
| K36 | | | | | 0,62 | | |
| | | | | | | | |
| **Farbe** | rötliches beige | helles rot | rötliches beige | orange | rötliches beige | bläuliches beige | orange |
| **Nach einer Woche** | warmes braun | rot | warmes braun | orange | helles blaurot | braunrot | rotblau |

**Tabelle 6:**

| **Beispiele 37 - 39** | | | |
|---|---|---|---|
| **Bsp.** | **37** | **38** | **39** |
| **Farbstoffe** | | | |
| E8 | 1,2 | 1,2 | 1,2 |
| K12 | | 0,63 | |
| K13 | 0,56 | | |
| K14 | | | 0,5 |
| K32 | 0,19 | 0,19 | 0,19 |
| K34 | 0,33 | 0,33 | 0,33 |
| | | | |
| **Farbe** | bläuliches beige | bläuliches beige | bläuliches beige |
| **Nach einer Woche** | warmes braun | warmes braun | warmes braun |

**Tabelle 7:**

| **Beispiele 40 - 46** | | | | | | | |
|---|---|---|---|---|---|---|---|
| **Bsp.** | **40** | **41** | **42** | **43** | **44** | **45** | **46** |
| **Farb- stoffe** | | | | | | | |
| E1 | 4,5 | 0,05 | 0,9 | 0,8 | 0,81 | 0,8 | 0,8 |
| E8 | 0,05 | 2,24 | 1,2 | 1,12 | 1 | 1,26 | 0,7 |
| K11 | 0,55 | | | | | | |
| K15 | 0,35 | | | | | | |
| K17 | | | 2,01 | | | | |
| K19 | | | | | | | 2,6 |
| K18 | | | | | 0,3 | | |
| K21 | | | | | 0,1 | | |
| K22 | | | 0,15 | | | 1,12 | |
| K23 | | 1,5 | | | | | |
| K24 | | | | 2,17 | | | |
| K26 | | | | | 1,11 | | |
| K31 | | | | 0,1 | | 0,1 | |
| K32 | | | 0,2 | | | | |
| K34 | | | | | | | 0,1 |
| K35 | 1,15 | | | | | | |
| K36 | | 0,35 | | | | | |
| D2 | | 0,55 | | | | | |
| **Farbe** | helles gelb | helles orange | helles grau | helles orange | helles blau-violett | helles orange | perlgrau |
| **Nach einer Woche** | olive-braun | orange | helles grau | rot-orange | helles blau-violett | rot-orange | aschbraun |

**Tabelle 8:**

| **Beispiele 47 - 53** | | | | | | | |
|---|---|---|---|---|---|---|---|
| **Bsp.** | **47** | **48** | **49** | **50** | **51** | **52** | **53** |
| **Farb- stoffe** | | | | | | | |
| E1 | 0,8 | 0,9 | 0,7 | 0,5 | 0,9 | 0,2 | 0,78 |
| E8 | 1 | 1,2 | 1,1 | 1 | 0,9 | 2 | 0,16 |
| K12 | | 2 | | | | | |
| K13 | | | | | 1,6 | | |
| K14 | | | | | 0,3 | | |
| K16 | | | | 0,1 | | | |
| K18 | 0,3 | | 0,7 | | | | |
| K21 | 0,1 | | | | | | |
| K22 | | 0,15 | | | | 1,14 | 0,9 |
| K23 | | | | 0,95 | | | |
| K25 | 0,8 | | | | | | 0,1 |
| K32 | | 0,2 | | | | | |
| K33 | | | 0,5 | | | | |
| | | | | | | | |
| **Farbe** | helles blau-violett | helles grau | helles orange | helles rotblau | beige | orange | beige |
| **Nach einer Woche** | helles blau-violett | helles grau | hell aubergine-farben | rotblau | purpur-braun | orange | warmes braun |

**Tabelle 9:**

| **Beispiele 54 - 56** | | | |
|---|---|---|---|
| **Bsp.** | **54** | **55** | **56** |
| **Farb- stoffe** | | | |
| E1 | 0,25 | 1,4 | 0,47 |
| E8 | 1,3 | 0,3 | 0,5 |
| K13 | 0,75 | | 0,2 |
| K22 | 0,15 | 0,5 | 0,15 |
| K25 | | 0,1 | 0,2 |
| K31 | 0,12 | 0,2 | 0,1 |
| K32 | 0,2 | 0,15 | |
| | | | |
| **Farbe** | helles grau | beige | hell aubergine-farben |
| **Nach einer Woche** | helles grau | warmes braun | hell aubergine-farben |

**Tabelle 10:**

| **Beispiele 57 - 63** | | | | | | | |
|---|---|---|---|---|---|---|---|
| **Bsp.** | **57** | **58** | **59** | **60** | **61** | **62** | **63** |
| **Farb- stoffe** | | | | | | | |
| E3 | 0,85 | 0,85 | 0,75 | 0,9 | 0,7 | 0,51 | 0,95 |
| E8 | 1,24 | 0,72 | 1,02 | 1,25 | 1,2 | 1,1 | 0,95 |
| K12 | | | | 2,5 | | | |
| K13 | | | | | | | 1,6 |
| K14 | | | | | | | 0,35 |
| K16 | | | | | | 0,13 | |
| K19 | | 2,7 | | | | | |
| K18 | | | 0,3 | | 0,72 | | |
| K21 | | | 0,12 | | | | |
| K22 | 1,2 | | | 0,2 | | | |
| K23 | | | | | | 0,92 | |
| K25 | | | 0,83 | | | | |
| K31 | 0,15 | | | | | | |
| K32 | | | | 0,22 | | | |
| K33 | | | | | 0,55 | | |
| K34 | | 0,15 | | | | | |
| | | | | | | | |
| **Farbe** | helles orange | perlgrau | helles blau-violett | helles grau | helles orange | helles rotblau | beige |
| **Nach einer Woche** | rot-orange | asch-braun | helles blau-violett | helles grau | helles Aubergine | rotblau | purpur-braun |

**Tabelle 11:**

| **Beispiele 64 - 70** | | | | | | | |
|---|---|---|---|---|---|---|---|
| **Bsp.** | **64** | **65** | **66** | **67** | **68** | **69** | **70** |
| **Farb- stoffe** | | | | | | | |
| E7 | 0,42 | 0,01 | 0,4 | 1 | 1,15 | 1 | 1 |
| E8 | 0,02 | 1,06 | 1,2 | 0,8 | 0,9 | 0,8 | 0,8 |
| K12 | 0,1 | | | | | | |
| K13 | | | | | 0,92 | | |
| K14 | | | 1,16 | | | | |
| K16 | | | | | | | 0,99 |
| K17 | | | | | 0,98 | | |
| K22 | | 0,55 | | | | | |
| K23 | | | | | | 0,85 | |
| K24 | | | | 1,29 | | | |
| K26 | | | 0,347 | | | | |
| K31 | | | | 0,12 | | 0,12 | |
| K32 | | | | | | | 0,3 |
| K35 | 0,15 | | | | | | |
| K36 | 0,01 | | | | | | |
| D1 | | 0,15 | | | | | |
| D2 | | 0,35 | | | | | |
| D3 | 0,25 | | | | | | |
| **Farbe** | gold-kupfer | orange | helles blaurot | orange | beige | helles orange | helles olive |
| **Nach einer Woche** | gold-kupfer | intensives orange | blaurot | gelb-orange | helles palisander | helles braunrot | olive |

**Tabelle 12:**

| **Beispiele 71 - 75** | | | | | |
|---|---|---|---|---|---|
| **Bsp.** | **71** | **72** | **73** | **74** | **75** |
| **Farb- stoffe** | | | | | |
| E7 | 0,98 | 1,27 | 0,4 | 1,15 | 0,15 |
| E8 | 0,75 | 0,98 | 1,2 | 0,9 | 1,25 |
| K12 | | | | 0,98 | |
| K13 | | | | 0,92 | |
| K14 | | | 1,16 | | |
| K19 | | | | | 0,2 |
| K18 | | | | | 0,93 |
| K21 | 0,53 | | | | |
| K22 | | 0,8 | | | |
| K23 | | 0,1 | | | |
| K25 | | | 0,25 | | |
| K33 | 0,2 | | | | |
| K34 | | 0,2 | | | |
| | | | | | |
| **Farbe** | helles orange | beige | helles blaurot | beige | blau-violett |
| **Nach einer Woche** | rot-beige | beige | blaurot | helles palisander | blau-violett |

**Tabelle 13:**

| **Beispiele 76 - 80** | | | | | |
|---|---|---|---|---|---|
| **Bsp.** | **76** | **77** | **78** | **79** | **80** |
| **Farb- stoffe** | | | | | |
| E3 | 2,79 | 0,022 | 0,095 | 0,22 | 0,69 |
| E2 | 1 | 0,015 | 0,4 | 0,29 | 0,1 |
| E8 | 0,05 | 2,24 | 1,25 | 1,12 | 1,02 |
| K11 | 0,56 | | | | |
| K15 | 0,36 | | | | |
| K17 | | | 2,02 | | |
| K18 | | | | | 0,35 |
| K21 | | | | | 0,15 |
| K22 | | | 0,155 | | |
| K23 | | 1,5 | | | |
| K24 | | | | 2,15 | |
| K26 | | | | | 1,1 |
| K31 | | | | 0,155 | |
| K32 | | | 0,2 | | |
| K35 | 1,15 | | | | |
| K36 | | 0,35 | | | |
| D2 | | 0,55 | | | |
| **Farbe** | hell-gelb | helles orange | hell-grau | orange | helles blau-viloett |
| **Nach einer Woche** | olive-braun | orange | hell-grau | gelb-orange | helles blau-viloett |

Alle in der vorliegenden Anmeldung verwendeten Prozentangaben stellen, soweit nicht anders angegeben, Gewichtsprozente dar.

## Patentansprüche

1. Mittel zum Färben von Keratinfasern, welches vor der Anwendung nicht mit einem Oxidationsmittel vermischt wird und **dadurch gekennzeichnet ist, dass** es frei von Mangansalzen ist und in einem geeigneten kosmetischen Träger eine in Gegenwart von Luftsauerstoff zur Färbung von Keratinfasern geeignete Kombination aus
(a) mindestens einem 4,5-Diaminopyrazol-Derivat der allgemeinen Formel (I) oder dessen physiologisch verträglichen Salzen, 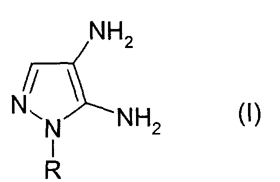 worin **R** Wasserstoff, eine geradkettige oder verzweigte Alkylgruppe mit 1 bis 6 Kohlenstoffatomen, eine geradkettige oder verzweigte Monohydroxyalkylgruppe mit 1 bis 6 Kohlenstoffatomen, eine geradkettige oder verzweigte Polyhydroxyalkylgruppe mit 2 bis 6 Kohlenstoffatomen, eine geradkettige oder verzweigte Monoaminoalkylgruppe mit 1 bis 6 Kohlenstoffatomen, eine geradkettige oder verzweigte Polyaminoalkylgruppe mit 2 bis 6 Kohlenstoffatomen, eine mit einem Halogenatom monosubstituierte Benzylgruppe, eine mit einer geradkettigen oder verzweigten Alkylgruppe mit 1 bis 6 Kohlenstoffatomen monosubstituierte Benzylgruppe, eine mit einer geradkettigen oder verzweigten Alkoxygruppe mit 1 bis 6 Kohlenstoffatomen monosubstituierte Benzylgruppe oder eine unsubstituierte Benzylgruppe darstellt; und
(b) mindestens einer Verbindung, welche ausgewählt ist aus
(b1) 5-Amino-2-methylphenolderivaten der allgemeinen Formel (II) oder deren physiologisch verträglichen Salzen, 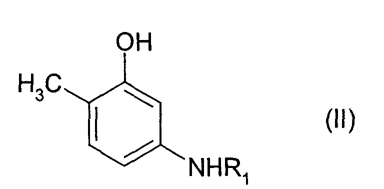 worin **R1** gleich Wasserstoff, einer geradkettigen oder verzweigten Alkylgruppe mit 1 bis 4 Kohlenstoffatomen, einer geradkettigen oder verzweigten Monohydroxyalkylgruppe mit 2 bis 4 Kohlenstoffatomen oder einer geradkettigen oder verzweigten Polyhydroxyalkylgruppe mit 3 bis 4 Kohlenstoffatomen ist; und
(b2) m-Phenylendiaminderivaten der allgemeinen Formel (III) oder deren physiologisch verträglichen Salzen, 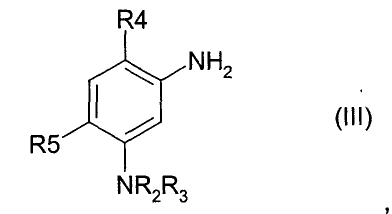 worin **R2** und **R3** unabhängig voneinander ein Wasserstoffatom, eine geradkettige oder verzweigte Alkylgruppe mit 1 bis 4 Kohlenstoffatomen, eine geradkettige oder verzweigte Monohydroxyalkylgruppe mit 2 bis 4 Kohlenstoffatomen oder eine geradkettige oder verzweigte Polyhydroxyalkylgruppe mit 3 bis 4 Kohlenstoffatomen bedeuten, und
**R4** und **R5** unabhängig voneinander ein Wasserstoffatom, eine geradkettige oder verzweigte Alkylgruppe mit 1 bis 6 Kohlenstoffatomen, eine geradkettige oder verzweigte Alkoxygruppe mit 1 bis 4 Kohlenstoffatomen, eine geradkettige oder verzweigte Monohydroxyalkoxygruppe mit 2 bis 4 Kohlenstoffatomen, eine geradkettige oder verzweigte Polyhydroxyalkoxygruppe mit 3 bis 4 Kohlenstoffatomen, eine geradkettige oder verzweigte Monohydroxyalkylgruppe mit 2 bis 4 Kohlenstoffatomen, eine geradkettige oder verzweigte Polyhydroxyalkylgruppe mit 3 bis 4 Kohlenstoffatomen oder eine 2,4-Diaminophenoxyalkoxy-Gruppe mit 1 bis 4 Kohlenstoffatomen in der Alkylkette bedeuten; enthält.

2. Mittel nach Anspruch 1, **dadurch gekennzeichnet, dass** die Verbindung der allgemeinen Formel (I) ausgewählt ist aus 4,5-Diamino-1-methyl-1H-pyrazol; 4,5-Diamino-1-(4'-methylbenzyl)-pyrazol; 4,5-Diamino-1 -(2'-hydroxyethyl)-1H-pyrazol; 4,5-Diamino-1-benzyl-1 H-pyrazol; 4,5-Diamino-1-ethyl-1H-pyrazol; 4,5-Diamino-1-isopropyl-1H-pyrazol; 4,5-Diamino-1-(4'-methoxybenzyl)-1 H-pyrazol; 4,5-Diamino-1H-pyrazol; 4,5-Diamino-1-(3'-methoxybenzyl)-1 H-pyrazol und 4,5-Diamino-1-(4'-chlorbenzyl)-1H-pyrazol, oder deren physiologisch verträglichen Salzen.

3. Mittel nach Anspruch 1, **dadurch gekennzeichnet, dass** die Verbindung der allgemeinen Formel (II) ausgewählt ist aus 5-Amino-2-methylphenol, 5-Methylamino-2-methylphenol und 5-[(2'-Hydroxyethyl)-amino]-2-methylphenol oder deren physiologisch verträglichen Salzen.

4. Mittel nach Anspruch 1, **dadurch gekennzeichnet, dass** die Verbindung der allgemeinen Formel (III) ausgewählt ist aus 2,4-Diamino-1-(2'-hydroxy-ethoxy)benzol, 2,4-Diamino-1-(2',3'-dihydroxypropoxy)-benzol, 1,3-Bis(2',4'-diaminophenoxy)propan, 2-Amino-4-[(2'-hydroxyethyl)-amino]anisol, 1,5-Bis(2'-hydroxyethoxy)-2,4-diaminobenzol und m-Phenylendiamin oder deren physiologisch verträglichen Salzen.

5. Mittel nach einem der Ansprüche 1 bis 4, **dadurch gekennzeichnet, dass** die Verbindungen der Formeln (I) bis (III) jeweils in einer Menge von 0,01 bis 5 Gewichtsprozent enthalten sind.

6. Mittel nach einem der Ansprüche 1 bis 5, **dadurch gekennzeichnet, dass** es zusätzlich weitere Oxidationsfarbstoffvorstufen enthält.

7. Mittel nach einem der Ansprüche 1 bis 6, **dadurch gekennzeichnet, dass** es zusätzlich einen oder mehrere direktziehende Farbstoffe aus der Gruppe der sauren oder basischen Farbstoffe, Triphenylmethanfarbstoffe, Anthrachinonfarbstoffe, aromatischen Nitrofarbstoffe, Azofarbstoffe, Lebensmittelfarbstoffe oder Dispersionsfarbstoffe enthält.

8. Mittel einem der Ansprüche 1 bis 7, **dadurch gekennzeichnet, dass** es einen pH-Wert von 6,5 bis 11,5 aufweist.

9. Verfahren zum Färben von Haaren, **dadurch gekennzeichnet, dass** man ein Haarfärbemittel nach einem der Ansprüche 1 bis 8 ohne vorheriges Vermischen mit einem Oxidationsmittel auf das Haar aufträgt, bei einer Temperatur von 15 bis 50°C 5 bis 45 Minuten lang einwirken lässt, das Haar anschliessend mit Wasser ausspült, gegebenenfalls shampooniert und sodann trocknet.

10. Verfahren nach Anspruch 9, **dadurch gekennzeichnet, dass** das Haarfärbemittel in einer Menge von 60 bis 200 Gramm verwendet wird.
